# EUROPEAN PATENT SPECIFICATION

(11) **EP 2 575 742 B1**
(45) Date of publication and mention of the grant of the patent: **02.11.2016**
(21) Application number: 11725954.9
(22) Date of filing: 24.05.2011
(51) Int. Cl.: A61K 8/23, A61K 8/34, A61K 8/42, A61K 8/46, A61K 8/49, A61K 8/66, A61K 8/81, A61Q 9/00, A61Q 9/04, A61K 8/04

(54) **IMPROVEMENTS IN OR RELATING TO DEPILATORY COMPOSITIONS**
VERBESSERUNGEN AN ODER IM ZUSAMMENHANG MIT DEPILATIONSZUSAMMENSETZUNGEN
AMÉLIORATIONS APPORTÉES OU ASSOCIÉES À DES COMPOSITIONS DÉPILATOIRES

(30) Priority: 03.06.2010 GB 201009275
(43) Date of publication of application: 10.04.2013
(73) Proprietor: Reckitt & Colman (Overseas) Limited, Slough Berkshire SL1 3UH (GB)
(72) Inventor: EVISON, Jane, East Yorkshire HU8 7DS (GB); RHOWBOTHAM, Sarah, East Yorkshire HU8 7DS (GB)
(74) Representative: O'Brien, Niall James
(86) International application number: PCT/GB2011/050977
(87) International publication number: WO 2011/151635

(56) References cited:
- EP-A2- 0 085 894
- EP-A2- 1 157 684
- WO-A1-2009/090362
- GB-A- 2 324 036
- US-A1- 2006 034 874
- US-B1- 6 231 846
- US-B1- 6 479 043
- KOPOLOW S L ET AL: "A NEW THICKENER/STABILIZER TECHNOLOGY", COSMETICS & TOILETRIES, WHEATON, IL, US, vol. 108, no. 5, 1 May 1993 (1993-05-01), pages 61-67, XP000982107, ISSN: 0361-4387

## Description

The present invention relates to a depilatory composition comprising a depilatory compound having a thiol group and to a method of depilation using such a composition. In particular, the present invention relates to depilatory gel having a thiol group but which suppresses the generation of malodour.

Compositions for removing superfluous body hair which include a compound that degrades hair keratin are well known. After the composition has been applied it is allowed to remain on the skin to degrade the hairs and is then removed together with the degraded hairs.

Depilatory compositions of this type typically comprise depilatory compounds which have a thiol group such as potassium thioglycolate. However, there is a significant disadvantage in using these compounds in that they have an unattractive smell. As a result, the composition will intrinsically have an unattractive smell which is noticed by a user when it is removed from a container. The reducing sulphur species present in the composition generates a variety of sulphur-containing volatiles, including hydrogen sulphide, methanethiol, dimethyl sulphide and dimethyl trisulphide, which are present at low levels in air above such products. During use, however, the unattractive smell can increase due to reaction of the depilatory compound with the hair which is being or which has been degraded. This is particularly unpleasant for the consumer. It would be desirable to have depilatory compositions with a reduced smell, in particular a reduced smell when in use.

Although agents are known to reduce the malodour when added to depilatory compositions, such agents also reduce the efficacy of the depilatory compound. An example of such an agent is zinc oxide.

Depilatory compositions may also typically contain compounds which can irritate and even damage the skin. For example they typically contain sodium hydroxide to provide a high pH.

According to the present invention there is provided a depilatory composition in the form of a gel which comprises a depilatory compound and at least 1.5 to 5 wt% based on the total weight of the composition of poly(methylvinyl ether/maleic anhydride) co-polymer crosslinked with 1,9 decadiene and wherein the depilatory compound may be any compound capable of degrading keratin selected from the group consisting of potassium thioglycolate, dithioerythritol, thioglycerol, thioglycol, thioxanthine, thiosalicylic acid, lipoic acid, sodium dihydrolipoate 6,8-dithiooctanoate, sodium 6,8-diothiooctanoate, a hydrogen sulphide salt, thioglycolic acid, 2-mercaptopropionic acid, 3-mercaptopropionic acid, thiomalic acid, ammonium thioglycolate, glyceryl monothioglycolate, monoethanolamine thioglycolate, monoethanolamine thioglycolic acid, diammoniumdithiodiglycolate, ammonium thiolactate, monoethanolamine thiolactate, thioglycolamide, homocysteine, cysteine, glutathione, dithiothreitol, dihydrolipoic acid, 1,3-dithiopropanol, thioglycolamide, glycerylmonothioglycolate, thioglycolhydrazine, keratinase, guanidine thioglycolate, calcium thioglycolate and/or cysteamine.

The cross-linked polymer is preferably present in the composition in an amount of from 2 to 3 wt%.

The depilatory composition further comprises a salt selected from di- and tri-valent salts, such as magnesium chloride, calcium chloride ammonium chloride, magnesium sulphate, calcium sulphate, aluminium sulphate, magnesium carbonate, calcium sulphate, calcium carbonate. A preferred salt is magnesium chloride. In an alternative embodiment the composition can comprise a salt extract, such as dead sea salts.

Preferably the depilatory compound is potassium thioglycolate.

The depilatory compound is preferably present in the composition in an amount of from 5 to 20wt% based on the total weight of the composition.

The depilatory composition may, if desired, comprise further components.

The depilatory composition may, for example, comprise components which accelerate the keratin degradation reaction such as urea, thiourea, dithioerythritol, dimethyl isosorbide (DMI), ethoxydiglycol (Transcutol) or methyl propyl diol (MT diol). The composition desirably comprises up to 15 wt% of the accelerator based on the total weight of the composition, preferably from 0.2 to 15 wt% and more preferably from 0.5 to 10 wt%.

The composition may comprise a source of alkalinity. This may include hydroxides, such as hydroxides of alkali and alkaline earth metals. Suitable hydroxides include sodium hydroxide, potassium hydroxide, calcium hydroxide and magnesium hydroxide. Preferably, potassium hydroxide is employed, optionally together with calcium hydroxide. The source of alkalinity (e.g. calcium hydroxide) may be present in an amount of 0.1 to 10 weight %, preferably 1 to 6 weight %, for example 2 to 5 weight % of the depilatory cream composition.

The depilatory cream composition preferably has a pH of greater than 7, for example, 9 to 12.7.

Preferably the alkali metal hydroxide is present in an amount of at least 0.001 mol/100 g of composition, preferably in an amount of at least 0.01 mol/100 g of the composition.

The depilatory composition of the present invention may comprise water, suitably in an amount of at least 40 or 50 wt% based on the total weight of the composition, more preferably at least 60 wt%.

The depilatory cream composition may further include a humectant. Suitable humectants include polyols, such as glycerine, propylene glycol and butylene glycol. Glycerine is preferred. The humectant may be present in an amount up to 10 wt %, preferably 0.5 to 5 wt %.

Other suitable components which may be incorporated into the composition include a buffer such as sodium silicate or magnesium silicate, fragrances such as lotus milk, and a chelating agent such as sodium gluconate.

The depilatory composition can comprise:
(a) 1.5 - 5% w/w poly(methylvinyl ether/maleic anhydride) co-polymer crosslinked with 1,9 decadiene;
(b) 0.01 - 1% w/w an inorganic salt;
(c) 0 - 10% w/w glycerine;
(d) 5 - 10% w/w urea;
(e) 0.01 - 1% w/w sodium gluconate;
(f) 0.01 - 1% w/w lotus milk extract;
(g) 10 - 15% w/w aqueous potassium thioglycolate solution;
(h) 1 - 5% w/w sodium silicate solution;
(i) 1 - 5% w/w potassium hydroxide; and
(j) water up to 100%.

In a preferred embodiment the depilatory composition can comprise:
(a) 2.2% w/w poly(methylvinyl ether/maleic anhydride) co-polymer crosslinked with 1,9 decadiene;
(b) 0.05% w/w magnesium chloride;
(c) 5 % w/w glycerine;
(d) 8 % w/w urea;
(e) 0.1% w/w sodium gluconate;
(f) 0.1% w/w lotus milk extract;
(g) 12% aqueous potassium thioglycolate solution;
(h) 2.5% w/w sodium silicate solution;
(i) 7.7% w/w potassium hydroxide 50% solution; and
(j) water up to 100%.

Example embodiments of the present invention will now be described.

**Example 1**

| **Ingredient (Trade Name)** | % w/w |
|---|---|
| Stabileze QM | 2.2 |
| Magnesium Chloride | 0.05 |
| Glycerine | 5 |
| Urea | 8 |
| Sodium Gluconate | 0.1 |
| Lotus Milk extract | 0.1 |
| Potassium thioglycolate solution (31 wt%) | 12 |
| Sodium Silicate solution | 2.5 |
| KOH 50% sol. | 7.7 |
| Deionised Water | 62.35 |
| **TOTAL** | **100** |

**Example 2**

| **Ingredient (Trade Name)** | **%w/w** |
|---|---|
| Stabileze QM | 2.5 |
| Glycerine | 5 |
| Urea | 8 |
| Sodium Gluconate | 0.1 |
| Lotus Milk extract | 0.1 |
| Potassium thioglycolate solution (31wt%) | 12.9 |
| Sodium Silicate solution | 2.5 |
| KOH 50% sol. | 8.2 |
| Deionised Water | 60.7 |
| **TOTAL** | **100** |

Stabileze QM is a cross-linked methylvinylether/maleic anhydride co-polymer.

The compositions of the Examples were compared with known gels to demonstrate the ability of the compositions of the present invention to reduce malodour when in contact with human hair. The gels which were used as a comparison contain different gelling or thickening agents. Typically, they contain acrylate/steareth-20 methacrylate co-polymer and/or carbomer.

The fragrance assessment was conducted in the following way. A dish is placed on a balance, and the balance is tared or 'zeroed'. 5g of gel is then added to the dish. The dish is placed in a bell jar, and the bell jar is closed to allow for any malodour generation to collect in the head-space of the bell jar. After 10 minutes a sniff test was conducted in the normal way. Each bell jar is covered in aluminium foil, to disguise the appearance of the gel to the volunteer.

The malodour assessment is conducted in the following way. A dish is placed on a balance, and the balance is tared or 'zeroed'. 0.025g of hair is added to the dish, and the dish is tared or 'zeroed' again. 5.0g of gel is then added to the dish. The hair and gel are mixed using a spatula as quickly as possible until homogenous. The dish is placed in a bell jar, and the bell jar is closed to allow for any malodour generation to collect in the head-space of the bell jar. After 10 minutes a sniff test was conducted in the normal way. Each bell jar was covered to disguise the appearance of the gel to the volunteer.

The volunteers were asked to rate the likeability of the fragrances and to rate the malodour coverage of the gel of the present invention and reference gel products. The volunteers removed the stopper, and first smelled two reference samples which were identical to the composition of Comparative Examples 1 & 2 - which they were asked to assign the number 10 for level of malodour. The Comparative Examples are products which are currently available.

The volunteers were then asked to smell the five test samples and mark each sample on the scale provided for presence of malodour.

The test sheets randomised the order in which the samples were tested. This randomisation was done using a Latin square technique.

Two repetitions were carried out. Each repetition involved 30 volunteers taking a sniff-test of the reference sample and then the three test samples, filling out the score sheet accordingly as they progressed. For each repetition fresh samples were made. The reference gel products both rated highly for their strength of malodour, and are therefore not rated as being suitable for counteracting malodour. They are also rated the lowest for freshness, strength of fragrance and the least pleasant. The gel of the present invention (both with and without fragrance) outperformed the reference gel products.

The graph below summarises the results which were obtained.

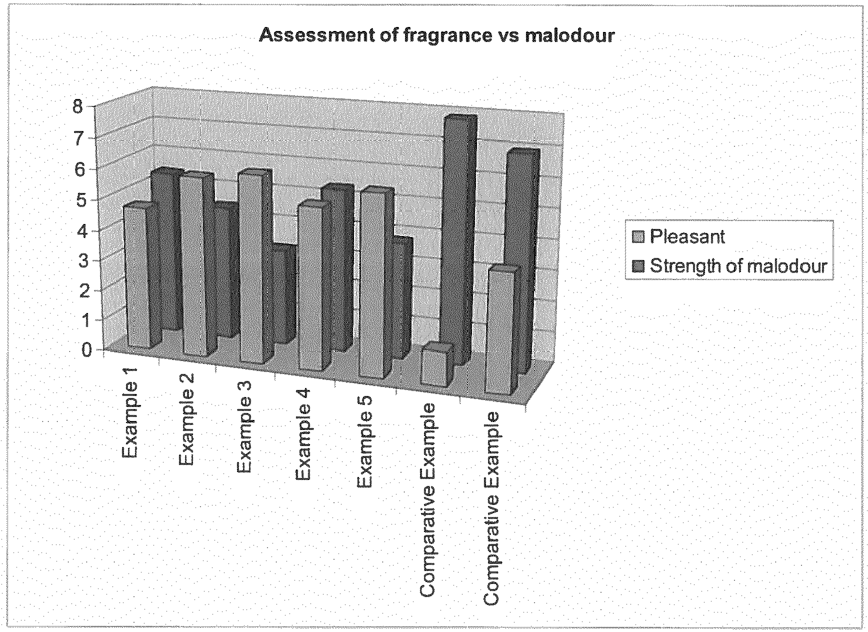

| **Fragrance** | **Pleasant** | **Strength of malodour** |
|---|---|---|
| Example 1 | 4.7 | 5.4 |
| Example 2 | 5.9 | 4.4 |
| Example 3 | 6.1 | 3.15 |
| Example 4 | 5.25 | 5.35 |
| Example 5 | 5.9 | 3.8 |
| Comparative Example | 1.1 | 7.85 |
| Comparative Example | 3.8 | 6.95 |

The examples of the present invention (Examples 1-5) only differed in the fragrance which was incorporated into the formulation. Example 1 did not include a fragrance, and therefore was the basic gel base, whereas examples 2 -5 contained a different fragrance. The Comparative Examples are products which are currently available.

An important feature of depilatory products containing thioglycolate is that they must exhibit good skin tolerance. This is due in part to the high pH that they work at (ca. pH 12.5). As a result, it important to ensure that such products do not exhibit high irritation levels.

The formulations of the present invention (examples 1 & 2 above) were, therefore, also tested for skin tolerance. Several different formulations based on these examples were also prepared. The formulations which included Stabileze QM (Ex. 1 & 2) exhibited good tolerance when compared to other gelling/thickening agents. The results are set out in the following table.

| **Thickener System** | **Tolerance** |
|---|---|
| Stabileze QM (Ex 1 & 2) | 0.49 |
| Aculyn and Structure 3001 | 2.09 |
| Ultrathix | 1.94 |
| Laponite and Structure 3001 | 1.24 |

In addition, the use of a salt such as magnesium chloride results in additional crosslinking/gellation of the composition which reduces the skin irritation and prevents the composition from transdermal adsorption. If there is less penetration or adsorption of the composition through the stratnum corneum there will less irritation. It is thought that glycolates may also weaken any thiol-thiol bonds within the skin.

The compositions can be formulated using standard techniques known to the man skilled in the art.

An advantage of the composition of the present invention is that there is provided a composition which reduces the unpleasant malodour experienced by an individual when using currently available compositions.

## Claims

1. A depilatory composition in the form of a gel which comprises a depilatory compound and at least 1.5 to 5 wt% based on the total weight of the composition of poly(methylvinyl ether/maleic anhydride) co-polymer crosslinked with 1,9 decadiene and wherein the depilatory compound may be any compound capable of degrading keratin selected from the group consisting of potassium thioglycolate, dithioerythritol, thioglycerol, thioglycol, thioxanthine, thiosalicylic acid, lipoic acid, sodium dihydrolipoate 6,8-dithiooctanoate, sodium 6,8-diothiooctanoate, a hydrogen sulphide salt, thioglycolic acid, 2-mercaptopropionic acid, 3-mercaptopropionic acid, thiomalic acid, ammonium thioglycolate, glyceryl monothioglycolate, monoethanolamine thioglycolate, monoethanolamine thioglycolic acid, diammoniumdithiodiglycolate, ammonium thiolactate, monoethanolamine thiolactate, thioglycolamide, homocysteine, cysteine, glutathione, dithiothreitol, dihydrolipoic acid, 1,3-dithiopropanol, thioglycolamide, glycerylmonothioglycolate, thioglycolhydrazine, keratinase, guanidine thioglycolate, calcium thioglycolate and/or cysteamine.

2. A depilatory composition as claimed in Claim 1 wherein the cross-linked polymer is present in the composition in an amount of from 2 to 3 wt% based on the total weight of the composition.

3. A depilatory composition as claimed in any of the preceding Claims wherein the composition further comprises a salt selected from di- and tri-valent salts, such as magnesium chloride, calcium chloride, ammonium chloride, magnesium sulphate, calcium sulphate, aluminium sulphate, magnesium carbonate, calcium sulphate, calcium carbonate.

4. A depilatory composition as claimed in Claim 3 wherein the salt is magnesium chloride.

5. A depilatory composition as claimed in Claim 1 wherein the depilatory compound is potassium thioglycolate.

6. A depilatory composition as claimed in any of the previous Claims wherein the depilatory compound is present in the composition in an amount of from 5 to 20 wt% based on the total weight of the composition.

7. A depilatory composition as claimed in any of the previous Claims wherein the depilatory compound is present in the composition in an amount of from 10 to 15 wt%.

8. A depilatory composition as claimed in any of the preceding Claims wherein the composition comprises a component which accelerates the keratin degradation reaction such as urea, thiourea, dithioerythritol, dimethyl isosorbide (DMI), ethoxydiglycol (Transcutol) or methyl propyl diol (MT diol).

9. A depilatory composition as claimed in Claim 8 wherein the composition comprises up to 15 wt% of the accelerator based on the total weight of the composition.

10. A depilatory composition as claimed in any of the preceding Claims comprising:
(a) 1.5 - 5% w/w poly(methylvinyl ether/maleic anhydride) co-polymer crosslinked with 1,9 decadiene;
(b) 0.01 - 1% w/w an inorganic salt;
(c) 0 - 10% w/w glycerine;
(d) 5 - 10% w/w urea;
(e) 0.01 - 1% w/w sodium gluconate;
(f) 0.01 - 1% w/w lotus milk extract;
(g) 10 15% w/w aqueous potassium thioglycolate solution;
(h) 1 - 5% w/w sodium silicate solution;
(i) 1 5% w/w potassium hydroxide; and
(j) water up to 100%.

11. A depilatory composition as claimed in Claim 10 wherein the composition comprises:
(a) 2.2% w/w poly(methylvinyl ether/maleic anhydride) co-polymer crosslinked with 1,9 decadiene;
(b) 0.05% w/w magnesium chloride;
(c) 5% w/w glycerine;
(d) 8% w/w urea;
(e) 0.1% w/w sodium gluconate;
(f) 0.1% w/w lotus milk extract;
(g) 12% w/w aqueous potassium thioglycolate solution;
(h) 2.5% w/w sodium silicate solution;
(i) 7.7% w/w potassium hydroxide 50% solution; and
(j) water up to 100%.

## Patentansprüche

1. Enthaarungszusammensetzung in der Form eines Gels, die eine Haarentfernungsverbindung und mindestens 1,5 bis 5 Gew.-%, bezogen auf das Gesamtgewicht der Zusammensetzung, von Poly(methylvinylether/Maleinsäureanhydrid)-Copolymer, das mit 1,9-Decadien vernetzt ist, umfasst, und wobei die Enthaarungsverbindung eine beliebige Verbindung sein kann, die Keratin abbauen kann, ausgewählt aus der Gruppe, bestehend aus Kaliumthioglycolat, Dithioerythrit, Thioglycerin, Thioglycol, Thioxanthin, Thiosalicylsäure, Liponsäure, Natriumdihydrolipoat-6,8-dithiooctanoat, Natrium-6,8-diothiooctanoat, ein Schwefelwasserstoffsalz, Thioglykolsäure, 2-Mercaptopropionsäure, 3-Mercaptopropionsäure, Thioäpfelsäure, Ammoniumthioglycolat, Glycerinmonothioglykolat, Monoethanolaminthioglycolat, Monoethanolaminthioglykolsäure, Diammoniumdithiodiglycolat, Ammoniumthiolactat, Monoethanolaminthiolactat, Thioglycolamid, Homocystein, Cystein, Glutathion, Dithiothreitol, Dihydroliponsäure, 1,3-Dithiopropanol, Thioglycolamid, Glycerylmonothioglycolat, Thioglycolhydrazin, Keratinase, Guanidinthioglycolat, Calciumthioglykolat und/oder Cysteamin.

2. Enthaarungszusammensetzung nach Anspruch 1, wobei das vernetzte Polymer in der Zusammensetzung in einer Menge von 2 bis 3 Gew.-%, bezogen auf das Gesamtgewicht der Zusammensetzung, vorliegt.

3. Enthaarungszusammensetzung nach einem der vorhergehenden Ansprüche, wobei die Zusammensetzung zudem ein Salz aus zwei- und dreiwertigen Salzen umfasst, wie Magnesiumchlorid, Calciumchlorid, Ammoniumchlorid, Magnesiumsulfat, Calciumsulfat, Aluminiumsulfat, Magnesiumcarbonat, Calciumsulfat, Calciumcarbonat.

4. Enthaarungszusammensetzung nach Anspruch 3, wobei das Salz Magnesiumchlorid ist.

5. Enthaarungszusammensetzung nach Anspruch 1, wobei die Enthaarungsverbindung Kaliumthioglycolat ist.

6. Enthaarungszusammensetzung nach einem der vorhergehenden Ansprüche, wobei die Enthaarungsverbindung in der Zusammensetzung in einer Menge von 5 bis 20 Gew.-%, bezogen auf das Gesamtgewicht der Zusammensetzung, vorhanden ist.

7. Enthaarungszusammensetzung nach einem der vorhergehenden Ansprüche, wobei die Enthaarungsverbindung in der Zusammensetzung in einer Menge von 10 bis 15 Gew.-% vorliegt.

8. Enthaarungszusammensetzung nach einem der vorhergehenden Ansprüche, wobei die Zusammensetzung eine Komponente umfasst, die die Keratin-Abbaureaktion beschleunigt, wie Harnstoff, Thioharnstoff, Dithioerythritol, Dimethylisosorbid (DMI), Ethoxydiglycol (Transcutol) oder Methylpropyldiol (MT Diol).

9. Enthaarungszusammensetzung nach Anspruch 8, wobei die Zusammensetzung bis zu 15 Gew.-% des Beschleunigers, bezogen auf das Gesamtgewicht der Zusammensetzung, umfasst.

10. Enthaarungszusammensetzung nach einem der vorhergehenden Ansprüche, umfassend:
(a) 1,5 - 5% w/w Poly(methylvinylether/Maleinsäureanhydrid)-Copolymer, vernetzt mit 1,9-Decadien;
(b) 0,01 - 1% w/w eines anorganischen Salzes;
(c) 0 - 10% w/w Glycerin;
(d) 5 - 10% w/w Harnstoff;
(e) 0,01 - 1% w/w Natriumgluconat;
(f) 0,01 - 1% w/w Lotusmilchextrakt;
(g) 10 - 15% w/w wässrige Kaliumthioglycolat-Lösung;
(h) 1 - 5% w/w Natriumsilikat-Lösung;
(i) 1 - 5% w/w Kaliumhydroxid; und
(j) Wasser auf 100%.

11. Enthaarungszusammensetzung nach Anspruch 10, wobei die Zusammensetzung Folgendes umfasst:
(a) 2,2% w/w Poly(methylvinylether/Maleinsäureanhydrid)-Copolymer, vernetzt mit 1,9-Decadien;
(b) 0,05% w/w Magnesiumchlorid;
(c) 5% w/w Glycerin;
(d) 8% w/w Harnstoff;
(e) 0,1% w/w Natriumgluconat;
(f) 0,1% w/w Lotusmilchextrakt;
(g) 12% w/w wässrige Kaliumthioglycolat-Lösung;
(h) 2,5% w/w Natriumsilikat-Lösung;
(i) 7,7% w/w Kaliumhydroxid 50% Lösung; und
(j) Wasser auf 100%.

## Revendications

1. Composition épilatoire sous la forme d'un gel qui comprend un composé épilatoire et au moins 1,5 à 5 % en poids sur la base du poids total de la composition de copolymère de poly(éther méthylvinylique/anhydride maléique) réticulé avec le 1,9-décadiène et le composé épilatoire pouvant être un composé quelconque capable de dégrader la kératine choisi dans le groupe constitué des thioglycolate de potassium, dithioérythritol, thioglycérol, thioglycol, thioxanthine, acide thiosalicylique, acide lipoïque, dihydrolipoate-6,8-dithiooctanoate de sodium, 6,8-diothiooctanoate de sodium, sel de sulfure d'hydrogène, acide thioglycolique, acide 2-mercaptopropionique, acide 3-mercaptopropionique, acide thiomalique, thioglycolate d'ammonium, monothioglycolate de glycéryle, thioglycolate de monoéthanolamine, acide monoéthanolamine-thioglycolique, dithiodiglycolate de diammonium, thiolactate d'ammonium, thiolactate de monoéthanolamine, thioglycolamide, homocystéine, cystéine, glutathion, dithiothréitol, acide dihydrolipoïque, 1,3-dithiopropanol, thioglycolamide, monothioglycolate de glycéryle, thioglycolhydrazine, kératinase, thioglycolate de guanidine, thioglycolate de calcium et/ou cystéamine.

2. Composition épilatoire selon la revendication 1 dans laquelle le polymère réticulé est présent dans la composition en une quantité de 2 à 3 % en poids sur la base du poids total de la composition.

3. Composition épilatoire selon l'une quelconque des revendications précédentes, la composition comprenant en outre un sel choisi parmi des sels di- et trivalents, tels que les chlorure de magnésium, chlorure de calcium, chlorure d'ammonium, sulfate de magnésium, sulfate de calcium, sulfate d'aluminium, carbonate de magnésium, sulfate de calcium, carbonate de calcium.

4. Composition épilatoire selon la revendication 3 dans laquelle le sel est le chlorure de magnésium.

5. Composition épilatoire selon la revendication 1 le composé épilatoire étant le thioglycolate de potassium.

6. Composition épilatoire selon l'une quelconque des revendications précédentes dans laquelle le composé épilatoire est présent dans la composition en une quantité de 5 à 20 % en poids sur la base du poids total de la composition.

7. Composition épilatoire selon l'une quelconque des revendications précédentes dans laquelle le composé épilatoire est présent dans la composition en une quantité de 10 à 15 % en poids.

8. Composition épilatoire selon l'une quelconque des revendications précédentes, la composition comprenant un composant qui accélère la réaction de dégradation de la kératine tel que l'urée, la thiourée, le dithioérythritol, le diméthylisosorbide (DMI), l'éthoxydiglycol (Transcutol) ou le méthylpropyldiol (MT diol).

9. Composition épilatoire selon la revendication 8, la composition comprenant jusqu'à 15 % en poids de l'accélérateur sur la base du poids total de la composition.

10. Composition épilatoire selon l'une quelconque des revendications précédentes comprenant :
(a) 1,5 à 5 % m/m de copolymère de poly(éther méthylvinylique/anhydride maléique) réticulé avec le 1,9-décadiène ;
(b) 0,01 à 1 % m/m d'un sel inorganique ;
(c) 0 à 10 % m/m de glycérine ;
(d) 5 à 10 % m/m d'urée ;
(e) 0,01 à 1 % m/m de gluconate de sodium ;
(f) 0,01 à 1 % m/m d'extrait de lait de lotus ;
(g) 10 à 15 % m/m de solution aqueuse de thioglycolate de potassium ;
(h) 1 à 5 % m/m de solution de silicate de sodium ;
(i) 1 à 5 % m/m d'hydroxyde de potassium ; et
(j) de l'eau jusqu'à 100 %.

11. Composition épilatoire selon la revendication 10, la composition comprenant :
(a) 2,2 % m/m de copolymère de poly(éther méthylvinylique/anhydride maléique) réticulé avec le 1,9-décadiène ;
(b) 0,05 % m/m de chlorure de magnésium ;
(c) 5 % m/m de glycérine ;
(d) 8 % m/m d'urée ;
(e) 0.1 % m/m de gluconate de sodium ;
(f) 0.1 % m/m d'extrait de lait de lotus ;
(g) 12 % m/m de solution aqueuse de thioglycolate de potassium ;
(h) 2,5 % m/m de solution de silicate de sodium ;
(i) 7,7 % m/m de solution à 50 % d'hydroxyde de potassium ; et
(j) de l'eau jusqu'à 100 %.
